# FASCICULE DE BREVET EUROPEEN

(11) **EP 0 567 536 B1**
(45) Date de publication et mention de la délivrance du brevet: **22.03.1995**
(21) Numéro de dépôt: 92903798.4
(22) Date de dépôt: 16.01.1992
(51) Int. Cl.: C12Q 1/30, C12Q 1/28, G01N 33/50, A61K 7/06

(54) **PROCEDE DE DIAGNOSTIC D'UN ETAT D'INFLAMMATION OU DE VIEILLISSEMENT CELLULAIRE DES KERATINOCYTES, ET NECESSAIRE POUR LA MISE EN OEUVRE DE CE PROCEDE**
VERFAHREN ZUR DIAGNOSTIK EINES STATUS EINER ENTZÜNDUNG ODER DER ZELLULÄREN ALTERUNG VON KERATINOZYTEN UND TESTBESTECK ZUR DURCHFÜHRUNG DES VERFAHRENS
METHOD FOR DIAGNOSING CELLULAR AGING OR INFLAMMATION CONDITION OF KERATINOCYTES, AND KIT FOR IMPLEMENTING SUCH METHOD

(30) Priorité: 16.01.1991 FR 9100457
(43) Date de publication de la demande: 03.11.1993
(73) Titulaire: L'OREAL, 75008 Paris (FR)
(72) Inventeur: PRUNIERAS, Michel, F-75015 Paris (FR); KERMICI, Michel, F-75012 Paris (FR); PRUCHE, Francis, F-75018 Paris (FR)
(74) Mandataire: Tonnellier, Jean-Claude
(86) Numéro de dépôt international: FR9200034
(87) Numéro de publication internationale: WO9213098

(56) Documents cités:
- MECHANISMS OF AGEING & DEVELOPMENT, vol. 53, no. 1, March 1990, Elsevier, Shannon (IE); M. KERMICI et al., pp. 73-84#

## Description

La présente invention a pour objet un procédé de diagnostic d'un état d'inflammation ou de vieillissement cellulaire des kératinocytes, ou de l'efficacité d'un traitement destiné à combattre ledit vieillissement cellulaire ou ladite inflammation.

On sait que l'épiderme est constitué de cellules appelées kératinocytes qui se renouvellent constamment par division au niveau de la couche basale et qui se différencient progressivement et évoluent pour former une couche de cellules kératinisées et donner finalement le Stratum Corneum, formé de cellules mortes constituant la couche la plus externe de l'épiderme.

On sait également que les cheveux sont produits dans des follicules pileux formés de gaines épithéliales d'origine épidermique et d'un bulbe (matrice) contenant des kératinocytes bulbaires en état de division permanent pendant les phases de croissance du cheveu (phase anagène), ces phases de croissance alternant avec des phases de repos au cours desquelles le cheveu cesse de croître et "tombe" alors naturellement. A la suite de cette chute naturelle du cheveu, la papille sous-jacente fabrique un nouveau follicule pileux, et le cycle recommence.

On constate, principalement chez les hommes, que dans la zone constituant le sommet du crâne (vertex), les cycles de production du cheveu s'arrêtent parfois précocement. C'est le phénomène de l'alopécie, qui n'est pas observé pour les zones du pourtour du crâne, et en particulier de la nuque.

On sait que ces phénomènes d'alopécie s'accompagnent de diverses modifications des kératinocytes du follicule pileux, en particulier de la teneur en glutathion ou de la teneur en glutathion peroxydase, qui sont en diminution chez les sujets ayant une alopécie établie; voir notamment M.Kermici et al. Congrès IFSCC, New York, Octobre 1990.

On a maintenant découvert que ces modifications, qui correspondent à un état de vieillissement cellulaire, sont déjà présenter chez les sujets non encore alopéciques, ou encore faiblement alopéciques. Il en va de même pour l'activité de catalase, présente dans les kératinocytes des follicules pileux, qui est augmentée dans les follicules pileux situés dans la "zone alopécique" des sujets alopéciques ou pré-alopéciques.

En dosant l'activité de ces divers marqueurs du vieillissement cellulaire, par comparaison avec la teneur en ces marqueurs des kératinocytes de follicule pileux situés dans les zones non alopéciques (nuque), il est possible de détecter les états pré-alopéciques permettant de prédire si un individu donné sera susceptible de "perdre ses cheveux" précocement.

On sait par ailleurs que l'alopécie peut être combattue notamment grâce à l'utilisation du Minoxidil. Cependant, le traitement au Minoxidil n'est pas efficace chez certains individus non répondeurs (environ 40 % des cas traités).

Le Minoxidil a notamment pour effet de rétablir l'équilibre des activités enzymatiques entre les follicules pileux du vertex et de la nuque. Cependant, ce rétablissement n'est observé que chez certains sujets (répondeurs) tandis que chez d'autres sujets, (non répondeurs), on n'observe pas un tel rétablissement.

Ces modifications de l'activité enzymatique, notamment chez les sujets prédisposés à l'alopécie, sont réversibles par le traitement avec le Minoxidil, généralement au bout de trois à six mois de traitement environ.

On a cependant découvert que cet effet souhaitable, à savoir notamment le rétablissement, par le traitement au Minoxidil, de l'équilibre de l'activité de glutathion peroxydase ou de la concentration de glutathion dans les follicules pileux de la zone du vertex, n'était possible que si cette activité ou cette concentration n'est pas déjà trop perturbée avant le traitement, car alors le rétablissement de l'équilibre par le Minoxidil ne peut plus être obtenu. Ainsi, lorsque le rapport des concentrations ou activités de ces marqueurs du vieillissement dans la zone testée et dans la zone témoin non pathologique, est supérieur ou, selon les cas, est inférieur à un certain seuil (qui peut être déterminé préalablement par des expériences de routine), il devient alors possible, grâce au procédé de l'invention, de prévoir si le traitement au Minoxidil sera efficace chez un individu donné.

Dans le cas de la catalase, au contraire, c'est lorsque l'activité de catalase est suffisamment augmentée, dans les follicules pileux du vertex par rapport à la nuque, que le traitement au Minoxidil sera efficace.

Cette possibilité de prédiction est intéressante car les traitements au Minoxidil sont longs et coûteux.

On a en outre découvert que des perturbations analogues, dans les teneurs des divers marqueurs mentionnés ci-dessus sont également présentes chez les kératinocytes de la peau, dans divers états pathologiques ou pré-pathologiques, ainsi que dans les états inflammatoires, ou encore dans des cas de vieillissement précoce des cellules de la peau non liés à une pathologie précise. Ici encore, en comparant la teneur en marqueurs du vieillissement cellulaire des kératinocytes de la zone testée avec celle des kératinocytes prélevés dans une zone de peau non pathologique, on obtient des informations permettant d'aider au diagnostic et de prescrire éventuellement un traitement adéquat. Le procédé de l'invention permet d'ailleurs également d'étudier les effets d'un tel traitement.

En effet, ce procédé permet de vérifier si ledit traitement conduit à un retour à l'équilibre des teneurs en marqueurs dans la zone de peau traitée.

Il convient encore de remarquer que, dans certains cas, la comparaison des teneurs en marqueurs des kératinocytes de la zone étudiée ou traitée pourra être comparée avec des valeurs moyennes relevées, après étude préalable, chez un nombre représentatif d'individus comparables représentant des échantillons de populations pathologiques et non pathologiques.

La présente invention a donc pour objet un procédé de diagnostic d'un état de vieillissement, cellulaire ou d'inflammation de kératinocytes chez un individu, ou de vérification de l'efficacité d'un traitement destiné à combattre ledit vieillissement ou ladite inflammation, caractérisé par le fait que, d'une part, on prélève un échantillon de kératinocytes dans une zone soupçonnée d'être pathologique (susceptible de souffrir d'un état de vieillissement cellulaire ou d'un état inflammatoire), ou dans la zone traitée, que d'autre part, on prélève des kératinocytes dans une zone témoin non pathologique, ou dans une zone non traitée, que l'on détermine, selon des méthodes connues en soi, les teneurs en un agent marqueur dudit état de vieillissement cellulaire ou dudit état d'inflammation dans lesdits kératinocytes et que l'on compare lesdites teneurs entre les kératinocytes de la zone pathologique avec celles de la zone saine ou de la zone non traitée, et par le fait que ledit marqueur est choisi parmi la catalase, le glutathion et la glutathion peroxydase, étant entendu que l'augmentation de l'activité de catalase, ou la diminution de la teneur en glutathion ou de l'activité de glutathion peroxydase pour les kératinocytes de la zone pathologique, ou de la zone traitée, par rapport aux kératinocytes de la zone non pathologique, ou de la zone non traitée, constitue le signe d'un état de vieillissement ou d'un état inflammatoire, ou d'une inefficacité du traitement.

On désigne généralement par l'expression "vieillissement cellulaire" un processus, parfois irréversible et parfois réversible, entraînant, en fonction du temps et/ou du nombre de divisions cellulaires, une modification biologique tissulaire ou cellulaire telle que par exemple une activité cellulaire réduite, une incapacité mitotique (perte de la capacité de division cellulaire), une perte de la capacité de synthèse de certaines protéines, ou encore la production de protéines (par exemple l'élastine) de mauvaise qualité. Le vieillissement cellulaire peut être induit ou accéléré par l'action de la lumière. On parle alors de photovieillissement.

Le procédé de l'invention peut bien entendu être mis en oeuvre non seulement sur des kératinocytes provenant d'un prélèvement de peau, mais aussi sur des cellules de la peau cultivées in vitro.

Selon un mode de réalisation du procédé, pour déterminer l'activité de catalase, on met en contact lesdits kératinocytes avec une quantité prédéterminée de peroxyde d'hydrogène ou d'hydroperoxyde, et on détermine à l'aide d'un système révélateur approprié si, après un temps prédéterminé (au bout duquel le révélateur, au contact des kératinocytes de la zone témoin, permet de mettre en évidence la présence de peroxyde d'hydrogène ou d'hydroperoxyde), le peroxyde d'hydrogène ou l'hydroperoxyde est présent, absent ou diminué dans les kératinocytes examinés, étant entendu que l'absence ou la diminution du peroxyde d'hydrogène ou de l'hydroperoxyde, par rapport aux kératinocytes de la zone témoin, est le signe d'une augmentation de l'activité de catalase, et inversement.

Selon un mode de réalisation particulier, pour déterminer l'activité de la catalase, on met en contact les kératinocytes avec un agent révélateur susceptible d'être décoloré par le peroxyde d'hydrogène ou par un hydroperoxyde que l'on ajoute une quantité prédéterminée de peroxyde d'hydrogène ou d'un hydroperoxyde, ou d'un système générateur de peroxyde d'hydrogène, et l'on détermine si au bout dudit temps prédéterminé, le réactif révélateur reste coloré, auquel cas l'activité de catalase dans les kératinocytes étudiés est augmentée, par rapport aux kératinocytes de la zone témoin, ce qui est le signe d'un état inflammatoire ou d'un état de vieillissement cellulaire, ou d'une inefficacité du traitement.

En particulier, pour déterminer l'activité de catalase, on met en contact lesdits kératinocytes avec un agent révélateur coloré susceptible d'être décoloré par le peroxyde d'hydrogène, et avec une oxydase et un substrat pour ladite oxydase, et on détermine si, après un temps prédéterminé, au bout duquel le réactif au contact des kératinocytes non pathologiques est décoloré, le réactif révélateur au contact des kératinocytes testés reste coloré, auquel cas l'activité de catalase dans les kératinocytes étudiés est augmentée, ce qui est le signe d'un état inflammatoire ou d'un état de vieillissement cellulaire, ou d'une inefficacité dudit traitement.

La présence de la catalase, ou de la glutathion peroxydase, dans le milieu biologique, peut être contrôlée par une détection immunologique à l'aide d'anticorps spécifiques, en particulier monoclonaux. La révélation des complexes formés avec ces anticorps peut être effectuée selon des méthodes de révélation immunologique classiques ou par le système décrit. Pour la mise en oeuvre de ces méthodes de détection immunologique, on peut citer par exemple YOCOTA, S. et FAKIMI, H.D. Immunocytochemical localization of catalase in rat liver, J. Histochem. Cytochem. 29 , 805-812 (1981), et J.A LITWIN et al., Immunocytochemical localization of peroxisomal enzymes in human liver biopsies, American Journal of Pathology vol 128, N° 1, (Juillet 1987).

Le système générateur de peroxyde d'hydrogène peut être constitué par toute oxydase produisant du peroxyde d'hydrogène en présence d'un substrat adéquat (par exemple glucose dans le cas de la glucose oxydase).

L'oxydase est par exemple la glucose oxydase ou la cholestétol oxydase.

L'agent révélateur est notamment constitué par une peroxydase et par un agent coloré susceptible d'être décoloré par le peroxyde d'hydrogène ou par un hydroperoxyde en présence d'une peroxydase.

L'agent coloré est par exemple le 2,6-dichloroindophénol.

Pour déterminer l'activité de la glutathion peroxydase, on met en contact lesdits kératinocytes avec une quantité prédéterminée de peroxyde d'hydrogène ou d'un hydroperoxyde, on ajoute du glutathion (formée réduite), de la glutathion réductase et son cofacteur (NADPH), et l'on mesure la quantité de NADPH consommée, qui est proportionnelle à l'activité de glutathion peroxydase.

Pour déterminer la teneur en glutathion, on met en contact les kératinocytes broyés avec un excès d'un agent oxydant capable de transformer le glutathion-forme réduite en glutathion-forme oxydée, et avec de la glutathion réductase et son cofacteur (NADPH), et on mesure soit la consommation de NADPH soit la quantité apparue de la forme réduite de l'oxydant.

Dans le procédé de détermination du glutathion, l'agent oxydant est par exemple l'acide dithiobis-2-nitrobenzoïque dont la forme réduite est l'acide 5-thio-2-nitrobenzoïque.

Les kératinocytes étudiés peuvent être ceux des bulbes pileux, en particulier ceux des cheveux. Selon ce mode de réalisation, on prélève les kératinocytes sous la forme d'un follicule pileux en phase anagène (obtenu par arrachage des cheveux).

Dans des modes de réalisation particuliers :
- on compare les résultats obtenus entre un follicule pileux prélevé sur une zone alopécique ou potentiellement alopécique, et un follicule pileux prélevé sur une zone non potentiellement alopécique (nuque).
- on calcule le rapport r de l'activité de catalase (c₁) présente dans le follicule prélevé sur le vertex a' l'activité de catalase (c₂) dans le follicule prélevé sur la nuque, et l'on détermine si ce rapport est supérieur à une valeur prédéterminée r₁, auquel cas si r est supérieur à r₁, on peut prédire que le traitement de l'alopécie par le Minoxidil, chez l'individu examiné, sera efficace tandis qu'il sera inefficace si r est inférieur à r₁ ; la valeur r₁ peut être déterminée par des expériences de routine ;
- on calcule le rapport r' de la concentration en glutathion ou de l'activité de glutathion peroxydase (c'₁) dans le follicule prélevé sur le vertex et la concentration en glutathion ou de l'activité de glutathion peroxydase (c'₂) dans le follicule prélevé sur la nuque, et l'on détermine si ce rapport r' est situé dans un intervalle prédéterminé expérimentalement, auquel cas le traitement de l'alopécie par le Minoxidil, chez l'individu examiné, sera efficace, et sera inefficace dans le cas contraire.

L'invention a également pour objet un nécessaire pour la mise enoeuvre du procédé décrit ci-dessus, caractérisé par le fait qu'il comprend :
- du peroxyde d'hydrogène, ou un hydroperoxyde, ou des moyens capables de produire du peroxyde d'hydrogène in situ, ou un agent oxydant capable de transformer le glutathion-forme réduite en glutathion-forme oxydée, et/ou un agent réducteur (NADPH) capable de transformer le glutathion-forme oxydée en glutathion-forme réduite ;
- des moyens pour déterminer la teneur ou l'activité en glutathion, glutathion peroxydase ou catalase ;
- et éventuellement des supports ou conteneurs pour ledit échantillon.

Dans des modes de réalisation particuliers, ledit nécessaire peut encore présenter les caractéristiques suivantes, prises isolément ou éventuellement, le cas échéant, en combinaison :
- lesdits moyens pour produire du peroxyde d'hydrogène in situ comprennent une oxydase.
- lesdits moyens pour déterminer l'activité de catalase comprennent un réactif révélateur coloré susceptible d'être décoloré par le peroxyde d'hydrogène ; ledit réactif révélateur coloré peut comprendre une peroxydase et un agent coloré susceptible d'être décoloré par le peroxyde d'hydrogène en présence d'une peroxydase ; ledit agent coloré et ladite peroxydase peuvent être déposés sur ledit support, sous la forme d'un film à base d'un matériau polymère tel que la gélatine ; ledit film peut contenir également une oxydase ;
- ledit nécessaire peut contenir en outre, dans un conteneur approprié, un substrat pour ladite oxydase ;
- ledit nécessaire peut contenir en outre, dans des conteneurs appropriés, du glutathion, de la glutathion réductase et du NADPH ;
- ou bien ledit nécessaire peut contenir en outre, dans des conteneurs appropriés, un agent oxydant capable de transformer le glutathion-forme réduite en glutathion-forme oxydée, de la glutathion réductase et du NADPH.

On va maintenant décrire les méthodes utilisées pour établir les résultats exposés ci-dessus qui sont à la base de l'invention.

### ABREVIATIONS

- GSH: Glutathion
- GSSG: Glutathion oxydé *
- GSH-PX: Glutathion Peroxydase *
- GSSG-RD: Glutathion Réductase *
- F.P.: Follicule pileux

* Activité enzymatique.

### MATERIELS et METHODES

### Les prélèvements

Les prélèvements ont été réalisés sur des sujets volontaires. Après arrachage d'une vingtaine de cheveux, l'observation à la loupe binoculaire permet la sélection des follicules pileux en phase anagène. L'homogénéisation est effectuée 24 heures après le prélèvement dans 100 »l de tampon PBS Dulbecco pH 7.4. Les activités sont déterminées directement sur l'homogénat.

C'est essentiellement sur les cellules de la gaine épithéliale externe d'origine épidermique que les dosages sont effectués.

### Dosage de la glutathion peroxydase (GSH-PX)

10 »l d'homogénat sont dilués dans 200 »l de tampon comprenant KH₂PO₄ 10 mM, EDTA 1 mM, NaN₃ 1 mM, NaCl 10 mM et glucose 5 mM, pH 7,0.

30 »l d'un mélange contenant du NADPH 0,2 mM, du GSH 1 mM, de la GSSG-RD 1 unité/ml sont ajoutés.

Après 5 minutes de préincubation à 37°C, 30 »l d'une solution de tert-butyl-hydroperoxyde 0,25 mM ou H₂O₂ 0,09 mM sont ajoutés pour initier la réaction. L'auto-oxydation du NADPH en présence de tert-butyl-hydroperoxyde est mesurée en parallèle, selon la méthode décrite par Paglia et Valentine, J. Lab. Clin. Med. (1967), 70, 158.

### Dosage du glutathion (oxydé et réduit)

Sur un aliquot de l'homogénat, les protéines sont précipitées par une solution d'acide 5-sulfosalicylique à 5 %. Après centrifugation, le dosage du GSH est effectué selon la méthode enzymatique de Griffith et Meister, P.N.A.S., USA (1979), 76, 268. Le GSH est oxydé par l'acide 5,5'-dithiobis-2 nitrobenzoïque (DTNB) avec formation stoechiométrique d'acide 5-thio-2-nitrobenzoïque (TNB). Le glutathion oxydé est réduit en GSH spécifiquement par la glutathion réductase en présence de NADPH. La formation de TNB est suivie spectrophotométriquement à 412 nm et est proportionnelle à la somme glutathion oxydé et réduit. En parallèle, une gamme de GSH est dosée dans les mêmes conditions (étalonnage). Les résultats sont exprimés en GSH/g d'ADN.

### Dosage de l'ADN par la méthode au DAPI(4-6-diamidino-2-phénylindole)

La méthode d'extraction de l'ADN et le dosage sont décrits par Meyer et Grudman, Arch. Dermatol. Res. (1984), 276, 52. Le dosage s'effectue sur 10 »l d'échantillon et le complexe fluorescent ADN-DAPI est déterminé avec λ exc 360 nm et λ ém 453 nm.

### Dosage de la CATALASE

Ce dosage est effectué selon la méthode décrite par Hugo Aebi in "Methods of Enzymatic Analysis" Hans Ulrich Bergmeyer Ed, Verlag Chemie Internat. Deersield Beach, Florida, Vol.2, pp.673-684 (1974).

Mesure de la diminution de H₂O₂ (pH : 7 à 25°C) à 240 nm ; (activité exprimée en »moles H₂O₂ détruites par minute et par »g d'ADN).

### EXEMPLES DE REALISATION D'UN TEST

On a réalisé un kit de diagnostic comprenant :
a) un système révélateur fixé dans un support de gélatine,
b) un réactif (glucose),
c) une échelle de référence adaptable à l'observation visuelle directe ou un système de lecture optique (photomètre, réfractomètre).

Le système colorant révélateur est le 2-6 dichloroindophénol. Ce colorant est utilisé a une concentration de 10 à 200 mg.l⁻¹.

La concentration usuelle est de 16 mg/l dans une solution de gélatine dans du tampon phosphate (pH = 7,4).

La gélatine contient des enzymes :
- la glucose oxydase (0,2 unité/ml)
- la peroxydase de raifort (0,16 unité/ml).

Le complexe gélatine-colorant-enzymes est étalé sur lame de verre et séché de façon à avoir un film homogène.

Le substrat de la glucose oxydase est du glucose (0,50 g/litre).

Le dosage s'effectue ainsi :
- Prélèvement d'un follicule pileux par arrachage près de la zone alopécique et/ou d'un follicule pileux par arrachage au niveau d'une zone non alopécique (nuque par exemple). Les follicules choisis sont en phase anagène.
- Le follicule pileux est déposé sur la lame de verre (ou un autre support) contenant le film gélatine-enzymes.

Une goutte de solution de glucose (20 »l) est déposée sur le follicule ainsi que sur une autre zone témoin.

La lecture s'effectue après 5 minutes à température ambiante (20 à 25°C).

La zone témoin s'est décolorée en totalité. Au niveau de la racine du follicule pileux, la décoloration est plus ou moins inhibée.

Dans le cas le plus simple, une référence à une gamme de bleu, et une gamme de taille calibrée, peut être faite grâce à un système de grossissement.

Moins la décoloration du 2-6 dichloroindophénol est importante, plus le risque alopécique est grand.

En effet, le système glucose oxydase/glucose produit "in situ" de l'H₂O₂. Celui-ci décolore le colorant (2-6 dichloroindophénol) en présence de peroxydase. Le follicule pileux, par l'activité catalase élevée, décompose l'H₂O₂ produit.

## Revendications

1. Procédé de diagnostic d'un état de vieillissement cellulaire ou d'inflammation de kératinocytes chez un individu, ou*de l'efficacité d'un traitement destiné à combattre ledit vieillissement ou ladite inflammation, caractérisé par le fait que, d'une part, on prélève un échantillon de kératinocytes dans une zone soupçonnée d'être pathologique (susceptible de souffrir d'un état de vieillissement cellulaire ou d'un état inflammatoire), ou dans la zone traitée, que d'autre part, on prélève des kératinocytes dans une zone témoin non pathologique, ou dans une zone non traitée, que l'on détermine, selon des méthodes connues en soi, les teneurs en un agent marqueur dudit état de vieillissement cellulaire ou dudit état d'inflammation dans lesdits kératinocytes, et que l'on compare lesdites teneurs entre les kératinocytes de la zone pathologique avec celles de la zone saine ou de la zone non traitée, et par le fait que ledit marqueur est choisi parmi la catalase, le glutathion et la glutathion peroxydase, étant entendu que l'augmentation de l'activité de catalase, ou la diminution de la teneur en glutathion ou de l'activité de glutathion peroxydase pour les kératinocytes de la zone pathologique, ou de la zone traitée, par rapport aux kératinocytes de la zone non pathologique, ou de la zone non traitée, constitue le signe d'un état de vieillissement ou d'un état inflammatoire, ou d'une inefficacité du traitement.

2. Procédé selon la revendication 1, caractérisé par le fait que, pour déterminer l'activité de catalase, on met en contact lesdits kératinocytes avec une quantité prédéterminée de peroxyde d'hydrogène ou d'hydroperoxyde, et on détermine à l'aide d'un système révélateur approprié si, après un temps prédéterminé (au bout duquel le révélateur, au contact des kératinocytes de la zone témoin, permet de mettre en évidence la présence de peroxyde d'hydrogène ou d'hydroperoxyde), le peroxyde d'hydrogène ou l'hydroperoxyde est présent, absent ou diminué dans les kératinocytes examinés, étant entendu que l'absence ou la diminution du peroxyde d'hydrogène ou de l'hydroperoxyde, par rapport aux kératinocytes de la zone témoin, est le signe d'une augmentation de l'activité de catalase, et inversement.

3. Procédé selon la revendication 2, caractérisé par le fait que, pour déterminer l'activité de catalase, on met en contact les kératinocytes avec un agent révélateur susceptible d'être décoloré par le peroxyde d'hydrogène ou par un hydroperoxyde, que l'on ajoute une quantité prédéterminée de peroxyde d'hydrogène ou d'un hydroperoxyde, et que l'on détermine si au bout dudit temps prédéterminé, le réactif révélateur reste coloré, auquel cas l'activité de catalase dans les kératinocytes étudiés est augmentée, par rapport aux kératinocytes de la zone témoin, ce qui est le signe d'un état inflammatoire ou d'un état de vieillissement cellulaire, ou d'une inefficacité du traitement.

4. Procédé selon la revendication 1, caractérisé par le fait que, pour déterminer l'activité de catalase, on met en contact lesdits kératinocytes avec un agent révélateur coloré susceptible d'être décoloré par le peroxyde d'hydrogène, et avec une oxydase et un substrat pour ladite oxydase, et on détermine si, après un temps prédéterminé, au bout duquel le réactif au contact des kératinocytes non pathologiques est décoloré, le réactif révélateur au contact des kératinocytes testés reste coloré, auquel cas l'activité de catalase dans les kératinocytes étudiés est augmentée, ce qui est le signe d'un état inflammatoire ou d'un état de vieillissement cellulaire, ou d'une inefficacité dudit traitement.

5. Procédé selon la revendication 4, caractérisé par le fait que ladite oxydase est la glucose oxydase.

6. Procédé selon l'une quelconque des revendications 3 à 5, caractérisé par le fait que ledit système révélateur est constitué par une peroxydase et par un agent coloré susceptible d'être décoloré par le peroxyde d'hydrogène ou par un hydroperoxyde en présence d'une peroxydase.

7. Procédé selon la revendication 6, caractérisé par le fait que l'agent coloré est le 2,6-dichloroindophénol.

8. Procédé selon la revendication 1, caractérisé par le fait que pour déterminer l'activité de la glutathion peroxydase, on met en contact lesdits kératinocytes avec une quantité prédéterminée de peroxyde d'hydrogène ou d'un hydroperoxyde, on ajoute du glutathion (forme réduite), de la glutathion réductase et son cofacteur (NADPH), et l'on mesure la quantité de NADPH consommée, qui est proportionnelle à l'activité de glutathion peroxydase.

9. Procédé selon la revendication 1, caractérisé par le fait que pour déterminer la teneur en glutathion, on met en contact les kératinocytes broyés avec un excès d'un agent oxydant capable de transformer le glutathion-forme réduite en glutathion-forme oxydée, et avec de la glutathion réductase et son cofacteur (NADPH), et que l'on mesure soit la consommation de NADPH soit la quantité apparue de la forme réduite de l'oxydant.

10. Procédé selon la revendication 9, caractérisé par le fait que ledit agent oxydant est l' acide dithiobis-2-nitrobenzoïque dont la forme réduite est l'acide 5-thio-2-nitrobenzoïque.

11. Procédé selon l'une quelconque des revendications 2, 3 et 6 à 8, caractérisé par le fait que ledit hydroperoxyde est choisi parmi le tert-butyl hydroperoxyde, le cumène hydroperoxyde, ou un hydroperoxyde lipidique éventuellement présent dans l'échantillon de kératinocytes.

12. Procédé selon l'une quelconque des revendications précédentes, caractérisé par le fait que lesdits kératinocytes sont prélevés sous la forme d'un follicule pileux en phase anagène.

13. Procédé selon la revendication 12, caractérisé par le fait que l'on compare les résultats obtenus entre un follicule pileux prélevé sur une zone alopécique ou potentiellement alopécique (vertex), et un follicule pileux prélevé sur une zone non potentiellement alopécique (nuque).

14. Procédé selon la revendication 13, caractérisé par le fait que l'on calcule le rapport r de l'activité de catalase (c₁) présente dans le follicule prélevé sur le vertex, à l'activité de catalase (c₂) dans le follicule prélevé sur la nuque, et l'on détermine si ce rapport est supérieur à une valeur prédéterminée r₁, auquel cas si r est supérieur à r₁, on peut prédire que le traitement de l'alopécie par le Minoxidil, chez l'individu examiné, sera efficace tandis qu'il sera inefficace si r est inférieur à r₁.

15. Procédé selon la revendication 8 ou 9, caractérisé par le fait que l'on calcule le rapport r' de la concentration en glutathion ou de l'activité de glutathion peroxydase (c'₁) dans le follicule prélevé sur le vertex à la concentration en glutathion ou l'activité de glutathion peroxydase (c'₂) dans le follicule prélevé sur la nuque, et l'on détermine si ce rapport r' est situé dans un intervalle prédéterminé expérimentalement, auquel cas le traitement de l'alopécie par la Minoxidil, chez l'individu examiné sera efficace, et sera inefficace dans le cas contraire.

16. Nécessaire pour la mise en oeuvre du procédé selon l'une quelconque des revendications précédentes, caractérisé par le fait qu'il comprend :
- du peroxyde d'hydrogène, ou un hydroperoxyde, ou des moyens capables de produire du peroxyde d'hydrogène in situ, ou un agent oxydant capable de transformer le glutathion-forme réduite en glutathion-forme oxydée, et/ou un agent réducteur capable de réduire le glutathion-forme oxydée en glutathion-forme réduite ;
- des moyens pour déterminer la teneur ou l'activité en glutathion, glutathion peroxydase ou catalase ;
- et éventuellement des supports ou conteneurs pour ledit échantillon.

17. Nécessaire selon la revendication 16, caractérisé par le fait que lesdits moyens pour produire du peroxyde d'hydrogène in situ comprennent une oxydase.

18. Nécessaire selon l'une quelconque des revendications 16 et 17, caractérisé par le fait que lesdits moyens pour déterminer l'activité de catalase comprennent un réactif révélateur coloré susceptible d'être décoloré par le peroxyde d'hydrogène.

19. Nécessaire selon la revendication 18, caractérisé par le fait que ledit réactif révélateur coloré comprend une peroxydase et un agent coloré susceptible d'être décoloré par le peroxyde d'hydrogène en présence d'une peroxydase.

20. Nécessaire selon la revendication 19, caractérisé par le fait que ledit agent coloré et ladite peroxydase sont déposés sur ledit support, sous la forme d'un film à base d'un matériau polymère tel que la gélatine.

21. Nécessaire selon la revendication 20, caractérisé par le fait que ledit film contient également une oxydase.

22. Nécessaire selon l'une quelconque des revendications 17 à 21, caractérisé par le fait qu'il contient en outre, dans un conteneur approprié, un substrat pour ladite oxydase.

23. Nécessaire selon l'une quelconque des revendications 16 et 17, caractérisé par le fait qu'il contient en outre, dans des conteneurs appropriés, du glutathion, de la glutathion réductase et du NADPH.

24. Nécessaire selon la revendication 16, caractérisé par le fait qu'il contient en outre, dans des conteneurs appropriés, un agent oxydant capable de transformer le glutathion-forme réduite en glutathion-forme oxydée, de la gluthation réductase et du NADPH.

## Claims

1. Method of diagnosis of a state of cell aging or of inflammation of keratinocytes in an individual, or of verification of the efficacy of a treatment intended for combating the said aging or the said inflammation, characterized in that, first, a sample of keratinocytes is taken from an area suspected of being pathological (liable to suffer from a state of cell aging or from an inflammatory state) or from the treated area, in that, secondly, keratinocytes are taken from a non-pathological control area or from an untreated area, in that the contents of a marker agent for the said state of cell aging or the said state of inflammation in the said keratinocytes are determined according to methods known per se, and in that the said contents of the keratinocytes of the pathological area are compared with those of the healthy area or of the untreated area, and characterized in that the said marker is chosen from catalase, glutathione and glutathione peroxidase, on the understanding that the increase in catalase activity or the decrease in glutathione content or in glutathione peroxidase activity for the keratinocytes of the pathological area or of the treated area, relative to the keratinocytes of the non-pathological area or of the untreated area, constitutes the sign of a state of aging or of an inflammatory state, or of a lack of efficacy of the treatment.

2. Method according to Claim 1, characterized in that, to determine catalase activity, the said keratinocytes are brought into contact with a predetermined amount of hydrogen peroxide or of hydroperoxide and, using a suitable visualizing system, it is determined whether, after a predetermined time (at the end of which the visualizing system, in contact with the keratinocytes of the control area, enables the presence of hydrogen peroxide or of hydroperoxide to be detected), hydrogen peroxide or hydroperoxide is present, absent or decreased in the keratinocytes under examination, on the understanding that the absence or decrease in hydrogen peroxide or hydroperoxide relative to the keratinocytes of the control area is the sign of an increase in catalase activity, and vice versa.

3. Method according to Claim 2, characterized in that, to determine catalase activity, the keratinocytes are brought into contact with a visualizing agent capable of being decolorized by hydrogen peroxide or by a hydroperoxide, in that a predetermined amount of hydrogen peroxide or of a hydroperoxide is added, and in that it is determined whether, at the end of the said predetermined time, the visualizing reagent remains coloured, in which case the catalase activity in the keratinocytes under study is increased relative to the keratinocytes of the control area, which is the sign of an inflammatory state or of a state of cell aging, or of a lack of efficacy of the treatment.

4. Method according to Claim 1, characterized in that, to determine catalase activity, the said keratinocytes are brought into contact with a coloured visualizing agent capable of being decolorized by hydrogen peroxide, and with an oxidase and a substrate for the said oxidase, and it is determined whether, after a predetermined time, at the end of which the reagent in contact with the non-pathological keratinocytes is decolorized, the visualizing reagent in contact with the test keratinocytes remains coloured, in which case the catalase activity in the keratinocytes under study is increased, which is the sign of an inflammatory state or of a state of cell aging, or of a lack of efficacy of the said treatment.

5. Method according to Claim 4, characterized in that the said oxidase is glucose oxidase.

6. Method according to any one of Claims 3 to 5, characterized in that the said visualizing system consists of a peroxidase and of a coloured agent capable of being decolorized by hydrogen peroxide or by a hydroperoxide in the presence of a peroxidase.

7. Method according to Claim 6, characterized in that the coloured agent is 2,6-dichloroindophenol.

8. Method according to Claim 1, characterized in that, to determine glutathione peroxidase activity, the said keratinocytes are brought into contact with a predetermined amount of hydrogen peroxide or of a hydroperoxide, glutathione (reduced form), glutathione reductase and its cofactor (NADPH) are added, and the amount of NADPH consumed, which is proportional to the glutathione peroxidase activity, is measured.

9. Method according to Claim 1, characterized in that, to determine glutathione content, the ground keratinocytes are brought into contact with an excess of an oxidizing agent capable of converting glutathione reduced form to glutathione oxidized form, and with glutathione reductase and its cofactor (NADPH), and in that either NADPH consumption or the amount which has appeared of the reduced form of the oxidizing agent is measured.

10. Method according to Claim 9, characterized in that the said oxidizing agent is dithiobis(2-nitrobenzoic acid), the reduced form of which is 5-thio-2-nitrobenzoic acid.

11. Method according to any one of Claims 2, 3 and 6 to 8, characterized in that the said hydroperoxide is chosen from tert-butyl hydroperoxide, cumene hydroperoxide and a lipid hydroperoxide possibly present in the keratinocyte sample.

12. Method according to any one of the preceding claims, characterized in that the said keratinocytes are sampled in the form of a hair follicle in the anagen phase.

13. Method according to Claim 12, characterized in that the results obtained are compared between a hair follicle taken from an alopecic or potentially alopecic area (vertex) and a hair follicle taken from an area which is not potentially alopecic (nape of the neck).

14. Method according to Claim 13, characterized in that the ratio r of catalase activity (c₁) present in the follicle taken from the vertex to the catalase activity (c₂) in the follicle taken from the nape of the neck is calculated, and it is determined whether this ratio is greater than a predetermined value r₁, in which case, if r is greater than r₁, it may be predicted that the treatment of alopecia with minoxidil in the individual under examination will be effective, while it will be ineffective if r is smaller than r₁.

15. Method according to Claim 8 or 9, characterized in that the ratio r' of the glutathione concentration or glutathione peroxidase activity (c'₁) in the follicle taken from the vertex to the glutathione concentration or glutathione peroxidase activity (c'₂) in the follicle taken from the nape of the neck is calculated, and it is determined whether this ratio r' lies within an experimentally predetermined range, in which case the treatment of alopecia with minoxidil in the individual under examination will be effective, and will be ineffective in the opposite case.

16. Kit for implementing the method according to any one of the preceding claims, characterized in that it comprises:
- hydrogen peroxide, or a hydroperoxide, or means capable of producing hydrogen peroxide in situ, or an oxidizing agent capable of converting glutathione reduced form to glutathione oxidized form, and/or a reducing agent capable of reducing glutathione oxidized form to glutathione reduced form;
- means for determining the glutathione, glutathione peroxidase or catalase content or activity;
- and, where appropriate, supports or containers for the said sample.

17. Kit according to Claim 16, characterized in that the said means for producing hydrogen peroxide in situ comprise an oxidase.

18. Kit according to either of Claims 16 and 17, characterized in that the said means for determining catalase activity comprise a coloured visualizing reagent capable of being decolorized by hydrogen peroxide.

19. Kit according to Claim 18, characterized in that the said coloured visualizing reagent comprises a peroxidase and a coloured agent capable of being decolorized by hydrogen peroxide in the presence of a peroxidase.

20. Kit according to Claim 19, characterized in that the said coloured agent and the said peroxidase are deposited on the said support in the form of a film based on a polymeric material such as gelatin.

21. Kit according to Claim 20, characterized in that the said film also contains an oxidase.

22. Kit according to any one of Claims 17 to 21, characterized in that it contains, in addition, in a suitable container, a substrate for the said oxidase.

23. Kit according to either of Claims 16 and 17, characterized in that it contains, in addition, in suitable containers, glutathione, glutathione reductase and NADPH.

24. Kit according to Claim 16, characterized in that it contains, in addition, in suitable containers, an oxidizing agent capable of converting glutathione reduced form to glutathione oxidized form, glutathione reductase and NADPH.

## Patentansprüche

1. Verfahren zur Diagnose eines zellulären Alterungs- oder Entzündungszustandes von Keratinozyten oder zur Überprüfung der Wirksamkeit einer Behandlung bei einem Individuum, die zur Bekämpfung der vorstehend genannten Alterung oder Entzündung bestimmt ist, wobei das Verfahren dadurch gekennzeichnet, daß man einerseits eine Keratinozytenprobe aus einer im Krankheitsverdacht stehenden Zone (weiche eine Anfälligkeil gegenüber Alterung oder Entzündung aufweist) oder aus einer behandelten Zone und andererseits Keratinozyten aus einer gesunden Vergleichszone oder einer nichtbehandelten Zone entnimmt, wobei man mit Hilfe an sich bekannter Verfahren den Gehalt an einem Marker des zellulären Alterungs- oder Entzündungszustandes mißt und die Gehalte der Keratinozyten aus der pathologischen Zone mit denjenigen der Zellen aus der gesunden oder nichtbehandelten Zone vergleicht, wobei der Marker aus Katalase, Glutathion sowie Glutathionperoxidase ausgewählt ist und zu verstehen ist, daß der Anstieg der Katalaseaktivität oder die Verringerung des Glutathiongehalts oder der Glutathionperoxidaseaktivität der Keratinozyten in der erkrankten bzw. der behandelten Zone im Vergleich zu den Keratinozyten aus der nichterkrankten bzw. nichtbehandelten Zone ein Anzeichen für einen Alterungs- oder Entzündungszustand bzw. die Unwirksamkeit der Behandlung darstellen.

2. Verfahren gemäß Anspruch 1, dadurch gekennzeichnet, daß man zur Bestimmung der Katalaseaktivität die Keratinozyten mit einer vorbestimmten Menge Wasserstoffperoxid oder Hydroperoxid in Kontakt bringt, mit Hilfe eines geeigneten Nachweissystems nach einer definierten Zeit bestimmt (wobei es das Nachweisreagenz nach deren Ablauf im Kontakt mit den Keratinozyten aus der Vergleichszone gestattet, die Anwesenheit von Wasserstoffperoxid oder eines Hydroperoxids unter Beweis zu stellen), ob Wasserstoffperoxid oder ein Hydroperoxid vorhanden oder nicht vorhanden oder sein Gehalt in den untersuchten Keratinozyten verringert ist, wobei dann das Fehlen oder die Verringerung des Wasserstoffperoxids oder Hydroperoxids, bezogen auf die Keratinozyten aus der Vergleichszone, das Signal für die Steigerung der Katalaseaktivität und umgekehrt darstellt.

3. Verfahren gemäß Anspruch 2, dadurch gekennzeichnet, daß man zur Bestimmung der Katalaseaktivität die Keratinozyten mit einem Nachweisreagenz in Kontakt bringt, welches durch Wasserstoffperoxid oder Hydroperoxid entfärbt wird, wobei man eine definierte Menge Wasserstoffperoxid oder Hydroperoxid einsetzt und mißt, ob das Nachweisreagenz nach Ablauf der vorgegebenen Zeit seine Farbe beibehält, was in diesem Fall bedeutet, daß die Katalaseaktivität in den untersuchten Keratinozyten, bezogen auf die Keratinozyten der Vergleichszone, gesteigert ist, wobei eine Steigerung ein Signal für einen Entzündungs- oder zellulären Alterungszustand oder die Unwirksamkeit einer Behandlung darstellt.

4. Verfahren gemäß Anspruch 1, dadurch gekennzeichnet, daß man zur Bestimmung der Katalaseaktivität die besagten Keratinozyten mit einem farbigen Nachweisreagenz, welches durch Wasserstoffperoxid entfärbt werden kann, sowie mit einer Oxidase und einem Substrat dieser Oxidase in Kontakt bringt und nach einer definierten Zeit, nach deren Ablauf das Reagenz im Kontakt mit den nichtpathologischen Keratinozyten entfärbt ist, bestimmt, ob das Nachweisreagenz im Kontakt mit den getesteten Keratinozyten seine Farbe behält, was in diesem Fall bedeutet, daß die Katalaseaktivität in den untersuchten Keratinozyten erhöht ist, wobei eine Steigerung das Signal für einen Entzündungs- oder zellulären Alterungszustand oder die Unwirksamkeit der Behandlung darstellt.

5. Verfahren gemäß Anspruch 4, dadurch gekennzeichnet, daß die Oxidase Glukoseoxidase ist.

6. Verfahren gemäß einem der Ansprüche 3 bis 5, dadurch gekennzeichnet, daß das Nachweissystem aus einer Peroxidase und einem Farbindikator besteht, welcher in Anwesenheit einer Peroxidase durch Wasserstoffperoxid oder ein Hydroperoxid entfärbt werden kann.

7. Verfahren gemäß Anspruch 6, dadurch gekennzeichnet, daß der Farbindikator 2,6-Dichlorindophenol ist.

8. Verfahren gemäß Anspruch 1, dadurch gekennzeichnet, daß man zur Bestimmung der Aktivität der Glutathionperoxidase die Keratinozyten mit einer vorbestimmten Menge Wasserstoffperoxid oder Hydroperoxid in Kontakt bringt, Glutathion (in reduzierter Form), Glutathionreduktase und ihr Coenzym (NADPH) zugibt und die Menge an verbrauchtem NADPH mißt, welche der Aktivität der Glutathionperoxidase proportional ist.

9. Verfahren gemäß Anspruch 1, dadurch gekennzeichnet, daß man zur Bestimmung des Glutathiongehaltes die zerkleinerten Keratinozyten mit einem Überschuß an einem Oxidationsmittel, welches die reduzierte Form des Glutathions in dessen oxidierte Form umwandeln kann, sowie mit Glutathionperoxidase und ihrem Coenzym (NADPH) in Kontakt bringt, wonach man entweder den Verbrauch an NADPH oder die gebildete Menge an Oxidationsmittel in der reduzierten Form mißt.

10. Verfahren gemäß Anspruch 9, dadurch gekennzeichnet, daß das Oxidationsmittel Dithiobis-2-nitrobenzoesäure ist, wobei die reduzierte Form 5-Thio-2-nitrobenzoesäure darstellt.

11. Verfahren gemäß einem der Ansprüche 2, 3 und 6 bis 8, dadurch gekennzeichnet, daß das Hydroperoxid aus t-Butylhydroperoxid, Cumolhydroperoxid oder einem lipiden Hydroperoxid ausgewählt ist, welches gegebenenfalls in einer Keratinozytenprobe vorliegen kann.

12. Verfahren gemäß einem der vorstehenden Ansprüche, dadurch gekennzeichnet, daß die Keratinozyten in Form eines Haarfollikels in der Regenerationsanfangsphase entnommen werden.

13. Verfahren gemäß Anspruch 12, dadurch gekennzeichnet, daß man die erhaltenen Ergebnisse eines aus einer Haarausfallszone oder potentiellen Haarausfallszone (Scheitel) entnommenen Haarfollikels mit denjenigen eines aus einer Zone, in den kein Haarausfall möglich ist, entnommenen Follikels (Nacken) miteinander vergleicht.

14. Verfahren gemäß Anspruch 13, dadurch gekennzeichnet, daß man das Verhältnis r der Katalaseaktivität (c₁) des vom Scheitel entnommenen Follikels zur Katalaseaktivität (c₂) des am Nacken entnommenen Follikels berechnet und bestimmt, ob dieses Verhältnis über einem vorherbestimmten Wert r₁ liegt, was bedeutet, daß man dann, wenn r oberhalb von r₁ liegt, vorhersagen kann, ob bei dem untersuchten Individuum die Behandlung des Haarausfalls mit Minoxidil wirksam ist oder ob sie, wenn r unterhalb von r₁ liegt, unwirksam ist.

15. Verfahren gemäß Anspruch 8 oder 9, dadurch gekennzeichnet, daß man das Verhältnis r' der Glutathionkonzentration oder der Aktivität der Glutathionperoxidase (c'₁) des vom Scheitel entnommenen Follikels zur Glutathionkonzentration oder zur Aktivität der Glutathionperoxidase (c'₂) des aus Gesicht oder Nacken entnommenen Follikel berechnet und bestimmt, ob sich dieses Verhältnis r' unterhalb eines experimentell zuvor bestimmten Bereiches befindet, was in diesem Fall bedeutet, daß bei dem untersuchten Individuum die Behandlung des Haarausfalls mit Minoxidil wirksam oder im umgekehrten Fall unwirksam ist.

16. Diagnosekit zur Durchführung des Verfahrens gemäß einem der vorstehenden Ansprüche, dadurch gekennzeichnet, daß er
- Wasserstoffperoxid oder ein Hydroperoxid oder Mittel, welche Wasserstoff *in situ* bilden können, oder ein Oxidationsmittel, welches die reduzierte Glutathionform in die oxidierte Glutathionform umwandeln kann, und/oder ein Reduktionsmittel, welches die oxidierte Form des Glutathions zu dessen reduzierter Form reduzieren kann;
- Reagenzien zur Bestimmung der Aktivitäten von Glutathion, Glutathionperoxidase oder Katalase;
- gegebenenfalls Träger oder Behältnisse für die Proben
enthält.

17. Diagnosekit gemäß Anspruch 16, dadurch gekennzeichnet, daß die Mittel zur Bildung von Wasserstoffperoxid *in situ* eine Oxidase umfassen.

18. Diagnosekit gemäß einem der Ansprüche 16 und 17, dadurch gekennzeichnet, daß die Reagenzien zur Bestimmung der Katalaseaktivität ein farbiges Nachweisreagenz umfassen, welches durch Wasserstoffperoxid entfärbt werden kann.

19. Diagnosekit gemäß Anspruch 18, dadurch gekennzeichnet, daß das farbige Nachweisreagenz eine Peroxidase und einen Farbindikator umfaßt, welcher in Anwesenheit einer Peroxidase durch Wasserstoffperoxid entfärbt werden kann.

20. Diagnosekit gemäß Anspruch 19, dadurch gekennzeichnet, daß der Farbindikator und die Peroxidase in Form eines Films auf Basis eines Polymermaterials wie Gelatine auf dem Träger aufgebracht sind.

21. Diagnosekit gemäß Anspruch 20, dadurch gekennzeichnet, daß der Film auch noch eine Oxidase enthält.

22. Diagnosekit gemäß einem der Ansprüche 17 bis 21, dadurch gekennzeichnet, daß er außerdem in einem geeigneten Behältnis ein Substrat der Oxidase enthält.

23. Diagnosekit gemäß einem der Ansprüche 16 und 17, dadurch gekennzeichnet, daß er zusätzlich noch in geeigneten Behältnissen Glutathion, Glutathionreduktase und NADPH enthält.

24. Diagnosekit gemäß Anspruch 16, dadurch gekennzeichnet, daß er außerdem in geeigneten Behältnissen ein Oxidationsmittel, welches die reduzierte Glutathionform in die oxidierte Glutathionform umwandeln kann, sowie Glutathionreduktase und NADPH enthält.
